# FASCICULE DE BREVET EUROPEEN

(11) **EP 3 145 387 B1**
(45) Date de publication et mention de la délivrance du brevet: **20.11.2019**
(21) Numéro de dépôt: 15728070.2
(22) Date de dépôt: 19.05.2015
(51) Int. Cl.: A61B 3/02, A61B 3/024, A61B 3/032

(54) **MÉTHODE DE TEST VISUEL D'UN INDIVIDU**
VERFAHREN ZUR VISUELLEN PRÜFUNG EINES INDIVIDUUMS
METHOD OF VISUAL TESTING OF AN INDIVIDUAL

(30) Priorité: 20.05.2014 FR 1454545
(43) Date de publication de la demande: 29.03.2017
(73) Titulaire: Essilor International, 94220 Charenton-le-Pont (FR)
(72) Inventeur: BONNIN, Thierry, 94227 Charenton-le-Pont, Cedex (FR); HADDADI, Ahmed, 94227 Charenton-le-Pont, Cedex (FR); MARIE, Sarah, 94227 Charenton-le-Pont, Cedex (FR)
(74) Mandataire: Jacobacci Coralis Harle
(86) Numéro de dépôt international: PCT/FR2015/051313
(87) Numéro de publication internationale: WO 2015/177458

(56) Documents cités:
- EP-A2- 1 442 695
- WO-A1-96/34555
- GB-A- 2 267 159
- US-A- 5 270 750

## Description

La présente invention concerne de manière générale le domaine de l'optique ophtalmique.

Elle concerne plus particulièrement une méthode et un dispositif de test visuel d'un individu permettant une démonstration objective de la qualité de vision de cet individu.

### ARRIERE-PLAN TECHNOLOGIQUE

Lors d'une visite chez un opticien, un individu souhaitant évaluer sa qualité de vision, avec ou sans équipement ophtalmique de correction visuelle, expérimente généralement une méthode de test visuel, qui peut être considérée comme subjective en ce sens que la qualité de vision de l'individu n'est pas mise en évidence et qu'aucun résultat de mesure concret ne peut être présenté à l'individu.

Le document EP 1442695 décrit par exemple une méthode pour mesurer la sensibilité aux contrastes dans le champ visuel d'un individu.

En outre, le résultat des méthodes de test visuel habituellement employées ne donne aucune indication à l'opticien quant aux zones accessibles du champ visuel que l'individu voit d'un côté nettes et de l'autre côté floues.

### OBJET DE L'INVENTION

Afin de remédier à l'inconvénient précité de l'état de la technique, la présente invention propose une méthode de test visuel permettant une démonstration objective de la qualité de vision de l'individu et une délimitation de celle-ci dans son champ visuel.

Plus particulièrement, on propose selon l'invention une méthode de test visuel d'un individu portant un équipement ophtalmique de correction visuelle, ladite méthode comportant les étapes suivantes :
a) placer ledit individu à une distance d'observation prédéterminée d'une surface d'affichage à laquelle ledit individu observe, avec un port de tête naturel, ladite surface d'affichage,
b) afficher, sur ladite surface d'affichage, un test visuel comprenant au moins une image-test, et
c) déterminer, sur ladite surface d'affichage, une frontière de zones de vision optimale/dégradée de ladite image-test par l'individu dans la configuration résultant des étapes a) et b), ladite frontière délimitant dans un champ visuel dudit individu muni dudit équipement ophtalmique respectivement une zone de vision optimale et une zone de vision dégradée dudit champ visuel, pour lesquelles ledit individu indique voir de manière optimale, respectivement de manière dégradée, ladite image-test du test visuel au travers dudit équipement ophtalmique de correction visuelle,

- d) enregistrer un indicateur de frontière représentatif de ladite frontière de zones de vision optimale/dégradée.

Selon l'invention, les étapes a) à d) sont exécutées au moins une première fois, avec un individu muni d'un premier équipement ophtalmique de correction visuelle, pour déterminer une première frontière et enregistrer un premier indicateur de frontière, et l'individu étant réellement ou virtuellement muni d'un deuxième équipement ophtalmique de correction visuelle différent dudit premier équipement ophtalmique :
- on détermine, sur ladite surface d'affichage, une deuxième frontière de zone de vision optimale/dégradée de ladite image-test par ledit individu dans la configuration résultant des étapes a) et b), ladite deuxième frontière délimitant respectivement une deuxième zone de vision optimale et une deuxième zone de vision dégradée dudit champ visuel, pour lesquelles ledit individu réellement ou virtuellement muni du deuxième équipement ophtalmique indique ou est supposé voir de manière optimale, respectivement de manière dégradée, ladite image-test du test visuel,
- on enregistre un deuxième indicateur de frontière représentatif de ladite deuxième frontière de zones de vision optimale/dégradée,
- on compare ledit premier indicateur de frontière et ledit deuxième indicateur de frontière, et
- on déduit de la comparaison précédente une performance optique de l'individu dans les conditions d'observation des étapes a) et b).

La première étape est une étape de positionnement de l'individu qui permet de mettre ledit individu dans des conditions d'observation proches de celles qu'il pourrait adopter dans la vie de tous les jours.

La distance d'observation prédéterminée sélectionne le type de vision que la méthode de test visuel souhaite mesurer : vision de près, vision de loin, ou vision intermédiaire.

La deuxième étape est une étape d'affichage comprenant la présentation à l'individu d'une image-test, généralement formée d'un texte destiné à être lu par ledit individu, placé à la distance d'observation prédéterminée à la première étape.

Grâce à la méthode de test visuel selon l'invention, il est possible d'obtenir une information relative à la qualité de vision de l'individu à l'intérieur de son champ visuel.

Cette qualité de vision se distingue en la détermination, d'une part, d'une zone de vision optimale et, d'autre part, d'une zone de vision dégradée, ces deux zones étant délimitées par la frontière de zones de vision optimale/dégradée.

Dans la zone de vision optimale, l'individu déclare voir nette ladite image-test du test visuel, alors que dans la zone de vision dégradée l'individu déclare voir floue ladite image-test.

Le critère d'appréciation entre zone de vision optimale et zone de vision dégradée est laissé à l'appréciation de l'individu.

La frontière de zones de vision optimale/dégradée matérialise ainsi la qualité de vision dudit individu.

L'invention trouve une application particulièrement avantageuse dans l'évaluation de la qualité de vision - en vision de près - d'un individu portant un équipement ophtalmique de correction visuelle comprenant un ou deux verres progressifs.

D'autres caractéristiques non limitatives et avantageuses de la méthode de test visuel conforme à l'invention sont les suivantes :
- ledit indicateur de frontière correspond à la position et/ou à la forme de ladite frontière ;
- ladite deuxième frontière est déterminée en exécutant une deuxième fois les étapes a) à c) pour ledit individu muni d'un exemplaire réel dudit deuxième équipement ophtalmique ;
- ladite deuxième frontière est déterminée par simulation numérique du champ visuel dudit individu virtuellement muni dudit deuxième équipement ophtalmique et observant l'image-test dans les mêmes conditions que celles des étapes a) et b) ;
- ladite méthode de test visuel comporte une étape f), dans laquelle on affiche, sur une surface utile de la surface d'affichage correspondant à la zone de vision optimale du champ visuel de l'individu muni dudit équipement ophtalmique de correction visuelle, un test visuel complémentaire comprenant au moins une image-test complémentaire ;
- on affiche en superposition l'image-test, la première frontière et la deuxième frontière ;
- à l'étape c), ladite frontière est déterminée pour une position donnée de la tête de l'individu par rapport à la surface d'affichage ;
- à l'étape b), la position de l'image-test dudit test visuel sur ladite surface d'affichage est fonction de la position de la tête de l'individu par rapport à la surface d'affichage ;
- à l'étape c), ladite frontière de zones de vision optimale/dégradée est déterminée par l'individu qui trace ladite frontière sur ladite surface d'affichage.

### DESCRIPTION DETAILLEE D'UN EXEMPLE DE REALISATION

La description qui va suivre en regard des dessins annexés, donnés à titre d'exemples non limitatifs, fera bien comprendre en quoi consiste l'invention et comment elle peut être réalisée.

Sur les dessins annexés :
- la figure 1 est une vue schématique de profil d'un individu sans équipement ophtalmique observant, en vision de près et avec un port de tête naturel, un texte affiché sur une tablette tactile numérique ;
- la figure 2 est une vue schématique de face de la tablette tactile de la figure 1 sur laquelle est affichée en superposition une frontière de zone déterminée par l'individu ;
- la figure 3 est une vue schématique de profil de l'individu de la figure 1, muni de verres progressifs et observant, en vision de près au travers desdits verres progressifs et avec un port de tête naturel, le texte affiché sur la tablette tactile numérique de la figure 2 ;
- la figure 4 est une représentation schématique du champ visuel de l'individu de la figure 3 au travers de son équipement ophtalmique ;
- la figure 5 est une vue schématique dans laquelle sont superposés le texte affiché par la tablette dans les conditions de la figure 3 et le champ visuel de la figure 4 ;
- la figure 6 est une vue schématique du texte affiché sur la tablette tactile des figures 1 et 3 et sur laquelle sont affichées en superposition deux frontières de zones déterminées respectivement par l'individu dans les conditions des figures 1 et 3.

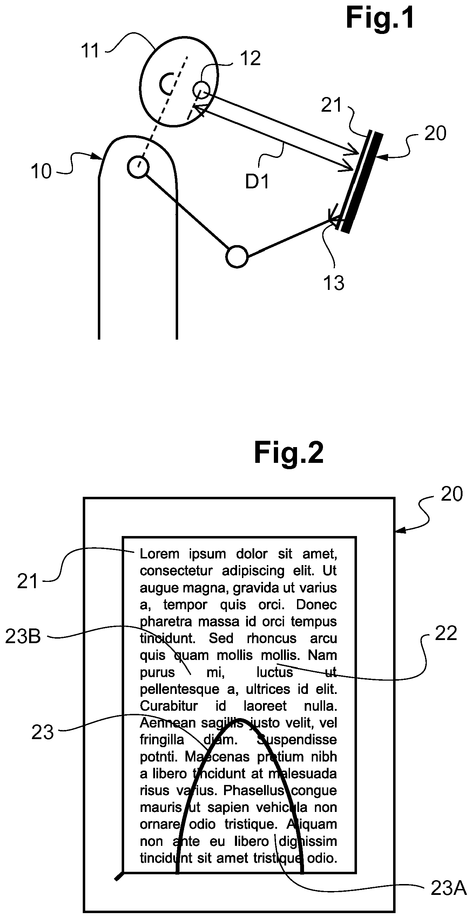

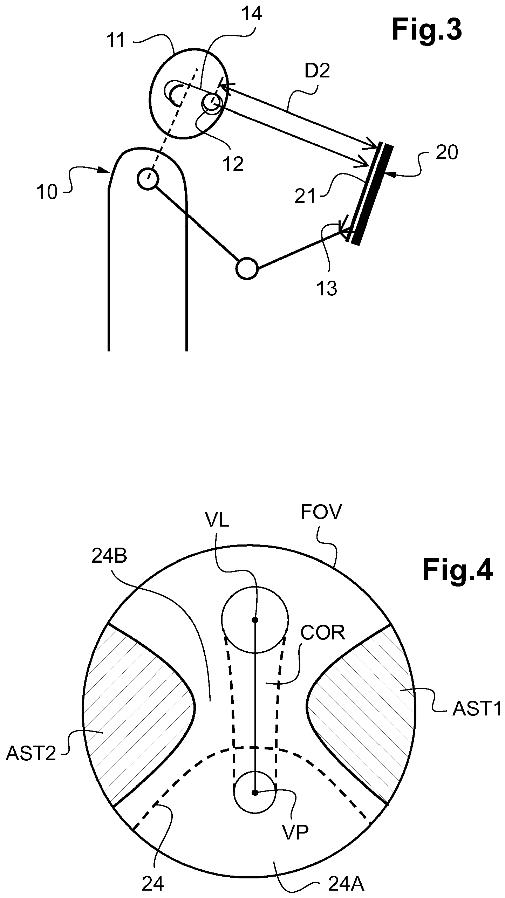

Les figures 1 et 3 représentent un individu 10 tenant entre ses mains un dispositif 20 de test visuel grâce auquel des méthodes de test visuel conforme à l'invention peuvent être mises en œuvre afin de tester la vision de cet individu 10.

On décrira, dans un premier temps, les moyens constitutifs de ce dispositif 20 de test visuel et, dans un second temps, différents exemples de méthode de test visuel pouvant être implémentés grâce à ce dispositif 20.

Sur les figures 1 et 3, seuls ont été représentés la tête 11, les yeux 12 et les mains 15 de l'individu 10 testé. Seul l'œil droit 12 de l'individu 10 est visible sur les figures 1 et 3.

Le dispositif 20 de test visuel comporte tout d'abord des moyens d'affichage. Dans l'exemple illustré, ces moyens d'affichage sont constitués par une tablette tactile.

En variante, les moyens d'affichage peuvent comprendre un moniteur, ou un projecteur associé à un écran sur lequel sont projetés des images ou du texte, ou tout simplement un support d'affichage classique tel qu'une feuille de papier.

La tablette 20 est conçue et programmée pour afficher sur une surface d'affichage, ici un écran 21 numérique, de préférence couleur, un test visuel.

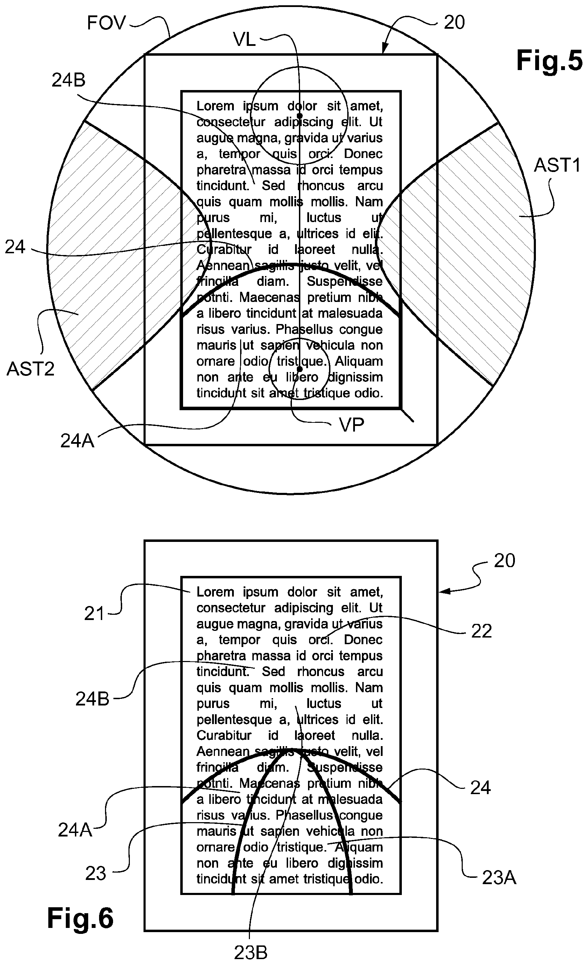

Dans les différents modes de réalisation de l'invention, le test visuel affiché sur l'écran 21 de la tablette 20 comprend au moins une image-test 22 (voir par exemple figures 2 et 6), cette image-test 22 étant de préférence aisément reconnaissable ou compréhensible par un individu moyen.

L'image-test 22 est de préférence une image classique, c'est-à-dire rencontrée dans la vie de tous les jours ou un texte formé de mots et de phrases facilement intelligible.

L'image-test 22 comprend ici un texte, par exemple un article de journal (voir figure 2 par exemple).

En variante, l'image-test peut comprendre des lettres, des mots, des chiffres ou des nombres, des images simples ou des motifs, par exemple des motifs géométriques, répétitifs ou non, agencés régulièrement ou non les uns par rapport aux autres.

L'écran 21 de la tablette 20 est, en condition de test, tourné vers l'individu 10 de sorte que celui-ci puisse visualiser le test visuel affiché sur cet écran 21 (voir figures 1 et 3).

Comme le montrent les figures 1 et 3, l'individu 10 observe l'écran 21 de la tablette 20 à une certaine distance d'observation, référencée D1 sur la figure 1 et D2 sur la figure 3.

Cette distance d'observation D1, D2 est prédéterminée en fonction de la nature du test visuel que l'on souhaite faire réaliser à l'individu 10.

En effet, selon que l'on souhaite tester la vision de près, la vision de loin ou la vision intermédiaire entre la vision de près et la vision de loin, on sélectionne une plage de distance d'observation adaptée.

Plus précisément, pour effectuer un test visuel destiné à évaluer la qualité de vision d'un individu :
- en vision de près : on choisit une distance d'observation inférieure à 40 centimètres environ.
- en vision de loin : on choisit une distance d'observation supérieure à 4 mètres environ, et
- en vision intermédiaire : on choisit une vision d'observation comprise entre 40 cm et 4 m.

Dans les différents modes de réalisation, les méthodes de test visuel décrites ci-après sont destinées à tester la vision de près, ou vision proche, de l'individu 10.

On va maintenant décrire en référence aux figures 1 et 2, un premier mode de réalisation d'une méthode de test visuel de l'individu 10 pouvant être mise en œuvre par un opticien pour évaluer la qualité de vision dudit individu 10 en vision de près et éventuellement prescrire une correction visuelle adaptée à cet individu 10.

Selon une première étape de la méthode de test visuel, l'opticien remet à l'individu 10 la tablette 20 sur l'écran 21 de laquelle l'image-test 22 est affichée.

Dans une deuxième étape, l'opticien place l'individu 10, ou bien demande à l'individu 10 de se placer, de manière à observer l'écran 21, avec un port de tête naturel, c'est-à-dire en adoptant une position de tête qui lui convient le mieux pour l'observation de l'écran 21.

En d'autres termes, lors de la deuxième étape, l'opticien prie l'individu 10 de regarder l'image-test 22 affichée sur l'écran 21, ici en tenant la tablette 20 à la distance d'observation D1 qui correspond à une distance d'observation en vision de près, par exemple égale à 40 centimètres.

Un des objectifs de cette étape est que l'individu 10 se trouve dans une situation proche de celle qu'il pourrait expérimenter dans son quotidien, en regardant des images ou lisant du texte en vision de près.

Dans la configuration résultant des première et deuxième étapes décrites ci-dessus, on détermine, dans une troisième étape de l'invention, une première frontière 23 sur l'écran 21 de la tablette 20 (voir figure 2).

Cette première frontière 23, qui est ici déterminée pour une position donnée de la tête 11 de l'individu 10 par rapport à l'écran 21 de la tablette 20, est définie comme étant une frontière de zones de vision optimale/dégradée de l'image-test 22 par l'individu 10, en ce sens qu'elle délimite dans le champ visuel de l'individu 10, d'une part, une zone de vision optimale 23A, et, d'autre part, une zone de vision dégradée 23B (voir figure 2).

Plus précisément, la détermination de la première frontière 23 de zones de vision optimale/dégradée peut être réalisée grâce au concours de l'opticien qui demande à l'individu 10 testé d'indiquer :
- la ou les zones de l'image-test 22 du test visuel pour lesquelles il voit de manière optimale : c'est le cas ici de la zone de vision optimale 23A, et
- la ou les zones de l'image-test 22 du test visuel pour lesquelles il voit de manière dégradée : c'est le cas ici de la zone de vision dégradée 23B.

Ici, pour l'image-test 22 utilisée et pour la distance d'observation D1 en vision de près, l'individu 10 déclare voir :
- net dans la zone de vision optimale 23A, et
- flou dans la zone de vision dégradée 23B.

La méthode de test visuel décrite ci-dessus permet notamment de tester soit l'hypermétropie d'un sujet jeune, soit la presbytie d'un sujet âgé, pour lesquels la vision de près est particulièrement dégradée.

Dans d'autres modes de réalisation de l'invention, l'individu détermine la frontière de zones de vision optimale/dégradée en fonction d'un autre critère de performance qui peut être, par exemple, le contraste de l'image-test, la distorsion des images ou des motifs dans l'image-test, les couleurs, ou tout autre critère que l'individu pourrait juger pertinent.

Afin de pouvoir matérialiser la première frontière 23, le dispositif de test visuel comporte des moyens de saisie de cette première frontière 23.

Dans les modes de réalisation représentés sur les figures 1 et 3 où le dispositif de test visuel comporte une tablette 20 tactile, les moyens de saisie comprennent notamment l'écran 21 de la tablette 20, sur lequel l'individu 10 peut tracer, par exemple au moyen d'un de ses doigts libres, la première frontière 23 sur l'écran 21.

Par « tracer », on entend ici que l'individu localise la première frontière par rapport à l'écran de la tablette.

Ainsi, en variante, l'individu pourrait tracer la première frontière sur l'écran au moyen d'un dispositif de pointage tel qu'une souris d'ordinateur ou une table traçante munie d'un stylo numérique.

En variante encore, l'individu pourrait indiquer oralement, par quels points de trajectoire passe la première frontière. Cette indication orale peut se faire à l'opticien ou bien à distance, directement ou indirectement.

La tablette 20 est ici conçue et programmée pour suivre la trajectoire tracée par l'individu 10 sur l'écran 21. La trajectoire suivie par le doigt libre de l'individu 10 sur l'écran 10 de la tablette 20 forme ainsi la première frontière 23 de zones de vision optimale/dégradée selon le critère choisi par l'individu 10.

De manière avantageuse, la tablette 20 peut également afficher, au fur et à mesure, et en même temps que le doigt libre de l'individu 10 se déplace sur l'écran 21, la première frontière 23.

En d'autres termes, le dispositif de test visuel formé ici par la tablette 20 comporte des moyens de traçage, ici l'écran 21, pour superposer l'image-test 22 et la première frontière 23 déterminée par l'individu 10 lors de la troisième étape de la méthode de test visuel.

À l'issue de la troisième étape, on a déterminé et affiché, pour une distance d'observation D1 prédéterminée pour la vision de près, une première frontière 23 sur l'écran 21 de la tablette 20, en superposition de l'image-test 22, cette première frontière 23 séparant une zone de vision optimale 23A de l'écran 21 où l'individu 10 voit net et une zone de vision dégradée 23B où l'individu 10 voit flou.

À partir de la première frontière 23 déterminée à la troisième étape, il est possible de déduire un premier indicateur de frontière relatif à cette première frontière 23.

Ce premier indicateur de frontière représente une donnée représentative de la qualité de vision de l'individu 10 mesurée par la méthode de test visuel.

Dans l'exemple de la figure 2, ce premier indicateur de frontière peut correspondre à la position de cette première frontière 23 par rapport à l'image-test 22 affichée sur l'écran ou encore à la forme de cette première frontière 23.

Ce premier indicateur de frontière peut également correspondre à l'aire (exprimée en centimètres carré par exemple) de la zone de vision optimale 23A, c'est-à-dire la surface sous la première frontière 23, ou à celle de la zone de vision dégradée 23B (surface au-dessus de la première frontière 23), ou encore au rapport de l'une de ces deux aires avec l'aire totale de l'image-test 22 ou l'aire totale de l'écran 21.

Ainsi, dans un mode de réalisation préféré, la méthode de test visuel comporte une quatrième étape consistant à enregistrer le premier indicateur de frontière.

À cet effet, le dispositif de test visuel comporte des moyens pour mémoriser cet indicateur.

Plus précisément ici, la tablette 20 est équipée d'un support de stockage numérique (non représenté) dans lequel la position et/ou la forme de la première frontière 23 sur l'écran 21 sont stockées.

Dans un autre mode de réalisation préféré, la méthode de test visuel comporte une cinquième étape qui consiste, dans un premier temps, à comparer l'indicateur de frontière déduit de la quatrième étape précédente à partir de la frontière de zones de vision optimale/dégradée déterminée sur l'écran de la tablette par l'individu avec un indicateur de référence.

Afin de permettre la comparaison, cet indicateur de référence est choisi en correspondance avec le premier indicateur de frontière. On entend par là que si le premier indicateur de frontière déterminé lors de la quatrième étape de la méthode de test visuel est relatif à la position et/ou à l'aire de la première frontière 23 correspondante, alors l'indicateur de référence est relatif à une position de référence et à une aire de référence. Cette situation est celle des figures 1 et 2.

Ainsi, la position et/ou l'aire de référence peuvent par exemple correspondre à une position et/ou une aire moyennes mesurées avec plusieurs individus, ou bien mesurées avec l'individu 10 de la figure 1 au cours de nombreux tests précédents.

Dans un second temps de la cinquième étape, on peut donc déduire de la comparaison précédente entre le premier indicateur de frontière et l'indicateur de référence une performance optique de l'individu 10 dans les conditions d'observation résultant des première et deuxième étapes.

En d'autres termes, la comparaison du premier indicateur de frontière avec l'indicateur de référence permet d'évaluer l'évolution de la position et/ou de l'aire de la première frontière 23 de zones de vision optimale/dégradée. Pour l'individu 10 de la figure 1, dans le cas où l'indicateur de référence comprend une position et/ou une aire moyennes comme vu précédemment, on peut ainsi déceler si la vision de près de l'individu 10 s'est dégradée ou non au cours du temps, lorsque par exemple l'aire de la zone de vision optimale 23A diminue. La performance optique testée et déduite de la comparaison entre le premier indicateur de frontière et l'indicateur de référence, correspond donc ici au champ visuel accessible en vision de près par l'individu 10.

Selon une variante de la méthode de test visuel décrite ci-dessus, on peut, dans une étape postérieure à la quatrième étape, déterminer à partir du premier indicateur de frontière enregistré sur le support de stockage numérique de la tablette 20 une conception optique recommandée d'un équipement ophtalmique recommandé de correction visuelle pour l'individu 10.

Selon cette variante, on détermine, à partir de la position et/ou de l'aire de la première frontière 23 sur l'écran 21 de la tablette 20, les puissances optiques des lentilles ophtalmiques de l'équipement ophtalmique recommandé pour l'individu 10.

À l'issue de cette étape, l'opticien est à même de conseiller à l'individu 10 une conception optique particulière d'équipement ophtalmique à même de corriger sa vision, ici sa vision de près.

Selon un deuxième mode de réalisation de la méthode de test visuel selon l'invention, qui sera décrit en référence aux figures 1 à 6, on exécute les première, deuxième, troisième étapes de la méthode telles que décrites précédemment avec l'individu 10, qui n'est pas ici muni d'un premier équipement de correction visuelle pour déterminer la première frontière 23 (troisième étape) et enregistrer le premier indicateur de frontière (quatrième étape) dans une mémoire numérique de la tablette 20.

Selon ce deuxième mode de réalisation, l'individu 10 est ici réellement muni d'un deuxième équipement ophtalmique 14 de correction visuelle (voir figure 3). Ce deuxième équipement ophtalmique 14 est typiquement une monture de lunettes comprenant deux lentilles ophtalmiques progressives pour la correction en vision de près et en vision de loin.

On considère ici que la conception optique de ce deuxième équipement ophtalmique 14 a été déterminée par l'opticien grâce à la première frontière 23 de zones de vision optimale/dégradée et grâce au premier indicateur de frontière.

Ce deuxième mode de réalisation de méthode de test visuel peut alors être avantageusement mis en œuvre pour le contrôle de ce deuxième équipement ophtalmique 14, par exemple lors de sa remise à l'individu 10 dont la qualité de vision a été précédemment testée.

Comme représenté sur la figure 3, lorsque l'individu 10 observe, avec un port de tête naturel, l'image-test 22 affichée sur l'écran 21 de la tablette 20, cette image-test est ici située à la distance d'observation D2 prédéterminée pour la vision de près. Cette distance d'observation D2 prédéterminée est ici égale à 35 cm.

L'individu 10 détermine, sur l'écran 21 de la tablette 20, une deuxième frontière 24 de zone de vision optimale/dégradée de l'image-test 22 par l'individu 10 (voir figure 6), dans la même configuration que les première et deuxième étapes mises en œuvre pour déterminer la première frontière 23.

De la même manière que précédemment, la deuxième frontière 24 délimite respectivement une deuxième zone de vision optimale 24A et une deuxième zone de vision dégradée 24B du champ visuel de l'individu 10, pour lesquelles l'individu 10, muni du deuxième équipement ophtalmique 14, indique voir de manière optimale, respectivement de manière dégradée, l'image-test 22 du test visuel affichée sur l'écran 21 de la tablette 20.

On a représenté sur la figure 4 une vue schématique du champ visuel FOV de l'individu 10 au travers de son deuxième équipement ophtalmique 14 comprenant des verres progressifs.

Sur cette figure 4 sont également représentés :
- le point VP de vision de près, situé de manière générale dans la partie basse du champ visuel de l'individu 10 porteur du deuxième équipement ophtalmique 14,
- le point VL de vision de loin, situé lui dans la partie haute du champ visuel, le point VL et le point VP définissant un corridor COR de correction visuelle le long duquel la puissance optique des verres progressifs varie (dans le sens des puissances plus élevées en se dirigeant vers le point VP), et
- les zones d'astigmatisme AST1, AST2 caractéristiques d'un verre progressif pour lesquelles la vision de l'individu 10 est dégradée à cause de l'astigmatisme des verres progressifs en bord du champ visuel FOV.

On a également représenté sur cette figure 4 la deuxième frontière 24 de zones de vision optimale/dégradée, cette deuxième frontière 24 étant donc ici relative à la vision de près de l'individu (voir configuration de la figure 3) et donc proche du point VP de vision de près qui fait d'ailleurs partie de la deuxième zone de vision optimale 24A alors que les zones d'astigmatisme AST1, AST2 (zones d'inconfort visuel) font, elles, partie de la zone de vision dégradée 24B que l'individu 10 indique voir de manière dégradée au travers du deuxième équipement ophtalmique 14.

Sur la figure 5, on a ainsi représenté en superposition, d'une part, l'image-test 22 affichée sur l'écran 21 de la tablette 20 et, d'autre part, le champ visuel FOV de l'individu 10, montrant ainsi quelles parties de l'image-test 22 sont vues de manière optimale et de manière dégradée dans le champ visuel FOV de l'individu 10.

Selon ce deuxième mode de réalisation, et comme le montre bien la figure 6, on affiche en superposition l'image-test 22, la première frontière 23 et la deuxième frontière 24 sur l'écran 21 de la tablette 20.

Par ailleurs, de la même manière que pour la première frontière 23, on détermine et on enregistre alors un deuxième indicateur de frontière qui est représentatif de la deuxième frontière 24 de zones de vision optimale/dégradée.

Ce deuxième indicateur de frontière comprend la position et la forme de la deuxième frontière 24 par rapport à l'image-test 22 affichée sur l'écran 21 de la tablette 20 (voir par exemple figure 5).

On compare ensuite le premier indicateur de frontière et le deuxième indicateur de frontière.

On compare par exemple, d'une part, les positions des première et deuxième frontières 23, 24 l'une par rapport à l'autre, et, d'autre part, les aires respectives des première et deuxième zones de vision optimale 23A, 24A ou dégradée 23B, 24B.

Comme cela est représenté sur la figure 6, on constate par exemple que l'individu 10 indique voir nette, avec le deuxième équipement ophtalmique 14, sur une deuxième zone de vison optimale 24A qui est plus étendue que la première zone de vision optimale 23A, déterminée sans équipement ophtalmique.

En superposant ainsi l'image-test 22, la première frontière 23 et la deuxième frontière 24, l'individu 10 peut se rendre compte du gain de champ visuel pour lequel il voit de manière optimale.

On déduit donc de la comparaison entre le premier indicateur de frontière et le deuxième indicateur de frontière, une performance optique de l'individu 10 dans les conditions d'observation des première et deuxième étapes, lorsque l'individu 10 observe l'image-test 22 affichée sur l'écran 21 de la tablette 20 à la distance d'observation D2 prédéterminée.

Dans un troisième mode de réalisation où, initialement, l'individu est muni réellement d'un équipement ophtalmique initial de correction visuelle, par exemple des verres progressifs comme ceux du deuxième équipement ophtalmique 12 de la figure 3, on peut déterminer une frontière initiale de zones de vision optimale/dégradée et enregistrer un indicateur initial de frontière de celle-ci grâce à l'individu observant, à la distance d'observation initiale prédéterminée, l'image-test affichée sur l'écran de la tablette.

Ensuite, l'individu étant réellement ou virtuellement muni d'un autre équipement ophtalmique de correction visuelle différent de cet équipement ophtalmique initial, on détermine, sur l'écran de la tablette, une autre frontière de zones de vision optimale/dégradée du champ visuel de l'individu.

Lorsque l'individu est virtuellement muni de l'autre équipement ophtalmique, cet autre frontière est alors déterminée par simulation numérique du champ visuel de l'individu observant l'image-test dans les mêmes conditions que celles des première et deuxième étapes, c'est-à-dire à la distance d'observation initiale et avec un port de tête naturel.

La simulation numérique du champ visuel de l'individu est effectuée à partir de la connaissance :
- de la position et de l'orientation de la tête de l'individu par rapport à l'écran de la tablette, lorsque l'individu adopte un port de tête naturel,
- de la distance d'observation initiale prédéterminée,
- de la correction visuelle de l'équipement ophtalmique initial, et
- de l'image-test affichée sur l'écran de la tablette.

De manière préférée, l'autre équipement ophtalmique de correction visuelle obtenu après simulation numérique est un équipement ophtalmique qui améliore l'indicateur de frontière déduit de la détermination de l'autre frontière de zones de vision optimale/dégradée. Par exemple, cet autre équipement ophtalmique pourra avantageusement agrandir la zone de vision optimale ou réduire la zone de vision dégradée que l'individu pourraient voir au travers de cet autre équipement ophtalmique.

Ce troisième mode de réalisation peut être avantageusement mis en œuvre par l'opticien souhaitant conseiller l'individu quant à un nouvel équipement ophtalmique, lorsque la qualité de vision d'un individu portant déjà réellement un équipement ophtalmique de correction visuelle a évolué.

Dans d'autres modes de réalisation, le dispositif de test visuel peut comprendre des moyens de repérage de la posture de la tête de l'individu par rapport à l'écran de la tablette.

De tels moyens de repérage peuvent comprendre par exemple un clip de repérage fixé sur l'équipement ophtalmique porté par l'individu comme celui décrit dans le document EP 2137569.

Dans ce cas, il est alors avantageux de déterminer la frontière de zones de vision optimale/dégradée avec une image-test du test visuel dont la position sur l'écran de la tablette est fonction de la position de la tête de l'individu par rapport à l'écran de la tablette.

Dans un autre mode de réalisation, la zone de vision optimale, où l'individu indique voir de manière optimale pour la distance d'observation prédéterminée avec son port de tête naturel, peut être mise à profit afin de réaliser des tests visuels complémentaires.

Ainsi, dans cet autre mode de réalisation, la méthode de test visuel comprend une sixième étape, dans laquelle on affiche, sur une surface utile de l'écran de la tablette correspondant à une zone utile comprise dans la zone de vision optimale du champ visuel de l'individu, un test visuel complémentaire comprenant une image-test complémentaire, qui sera donc vue de manière optimale par l'individu.

Ceci permet alors de se mettre dans les meilleures conditions afin d'effectuer le test visuel complémentaire.

Cela permet également d'afficher en superposition la frontière de zones de vision optimale/dégradée et un résultat obtenu à partir de ce test visuel complémentaire.

Dans les méthodes de test visuel où tester la vision intermédiaire l'individu 10 ou de loin est tester un dispositif de type manette commandée à distance ou pointeur sans fil pourront être utilisés pour tracer la frontière (23) de zones de vision optimale/dégradée de la dite image-test (22).

## Revendications

1. Méthode de test visuel d'un individu (10) portant un équipement ophtalmique de correction visuelle, ladite méthode comportant les étapes suivantes :
a) placer ledit individu (10) à une distance d'observation (D1) prédéterminée d'une surface d'affichage (21) à laquelle ledit individu (10) observe, avec un port de tête naturel, ladite surface d'affichage (21),
b) afficher, sur ladite surface d'affichage (21), un test visuel comprenant au moins une image-test (22),
c) déterminer, sur ladite surface d'affichage (21), une frontière (23) de zones de vision optimale/dégradée de ladite image-test (22) par l'individu (10) dans la configuration résultant des étapes a) et b), ladite frontière (23) délimitant dans un champ visuel dudit individu (10) muni dudit équipement ophtalmique respectivement une zone de vision optimale (23A) et une zone de vision dégradée (23B) dudit champ visuel, pour lesquelles ledit individu (10) indique voir de manière optimale, respectivement de manière dégradée, ladite image-test (22) du test visuel au travers dudit équipement ophtalmique de correction visuelle, et
d) enregistrer un indicateur de frontière représentatif de ladite frontière (23) de zones de vision optimale/dégradée,
selon laquelle :
- les étapes a) à d) sont exécutées au moins une première fois, avec un individu (10) muni d'un premier équipement ophtalmique de correction visuelle, pour déterminer une première frontière (23) et enregistrer un premier indicateur de frontière,
- l'individu (10) étant réellement ou virtuellement muni d'un deuxième équipement ophtalmique (14) de correction visuelle différent dudit premier équipement ophtalmique, on détermine, sur ladite surface d'affichage (21), une deuxième frontière (24) de zone de vision optimale/dégradée de ladite image-test (22) par ledit individu (10) dans la configuration résultant des étapes a) et b), ladite deuxième frontière (24) délimitant respectivement une deuxième zone de vision optimale (24A) et une deuxième zone de vision dégradée (24B) dudit champ visuel (FOV), pour lesquelles ledit individu (10) réellement ou virtuellement muni du deuxième équipement ophtalmique (14) indique ou est supposé voir de manière optimale, respectivement de manière dégradée, ladite image-test (22) du test visuel,
- on enregistre un deuxième indicateur de frontière représentatif de ladite deuxième frontière (24) de zones de vision optimale/dégradée,
- on compare ledit premier indicateur de frontière et ledit deuxième indicateur de frontière, et
- on déduit de la comparaison précédente une performance optique de l'individu (10) dans les conditions d'observation des étapes a) et b).

2. Méthode de test visuel selon la revendication 1, selon laquelle, à l'étape d), l'indicateur de frontière correspond à la position et/ou la forme de ladite frontière (23).

3. Méthode de test visuel selon la revendication 1 ou 2, selon laquelle ladite deuxième frontière (24) est déterminée en exécutant une deuxième fois les étapes a) à c) pour ledit individu (10) muni d'un exemplaire réel dudit deuxième équipement ophtalmique (14).

4. Méthode de test visuel selon la revendication 1 ou 2, selon laquelle ladite deuxième frontière (24) est déterminée par simulation numérique du champ visuel (FOV) dudit individu (10) virtuellement muni dudit deuxième équipement ophtalmique (14) et observant l'image-test (22) dans les mêmes conditions que celles des étapes a) et b).

5. Méthode de test visuel selon l'une des revendications 1 à 4, comportant une étape f), dans laquelle on affiche, sur une surface utile de la surface d'affichage (21) correspondant à la zone de vision optimale (23A) du champ visuel de l'individu (10) muni dudit équipement ophtalmique de correction visuelle, un test visuel complémentaire comprenant au moins une image-test complémentaire.

6. Méthode de test visuel selon l'une des revendications 1 à 4, selon laquelle on affiche en superposition l'image-test (22), la première frontière (23) et la deuxième frontière (24).

7. Méthode de test visuel selon l'une des revendications 1 à 6, selon laquelle, à l'étape c), ladite frontière (23,24) est déterminée pour une position donnée de la tête (11) de l'individu (10) par rapport à la surface d'affichage (21).

8. Méthode de test visuel selon l'une des revendications 1 à 7, selon laquelle, à l'étape b), la position de l'image-test (22) dudit test visuel sur ladite surface d'affichage (21) est fonction de la position de la tête (11) de l'individu (10) par rapport à la surface d'affichage (21).

9. Méthode de test visuel selon l'une des revendications 1 à 8, selon laquelle, à l'étape c), ladite frontière (23,24) de zones de vision optimale/dégradée est déterminée par l'individu (10) qui trace ladite frontière (23, 24) sur ladite surface d'affichage (21).

## Patentansprüche

1. Verfahren zum visuellen Testen einer Person (10), die eine ophthalmologische Apparatur zur Sehkorrektur trägt, wobei das Verfahren die folgenden Schritte aufweist:
a) Platzieren der Person (10) in einem vorbestimmten Betrachtungsabstand (D1) von einer Anzeigefläche (21), aus der die Person (10) die Anzeigefläche (21) mit einer natürlichen Kopfhaltung betrachtet,
b) Anzeigen eines visuellen Tests auf der Anzeigefläche (21), der mindestens ein Testbild aufweist (22),
c) Bestimmen einer Grenze (23) zwischen Zonen mit optimalem/verschlechtertem Sehen des Testbildes (22) durch die Person (10) auf der Anzeigefläche (21) in der Konfiguration, die sich aus den Schritten a) und b) ergibt, wobei die Grenze (23) in einem Sichtfeld der Person (10), die mit der ophthalmologischen Apparatur ausgestattet ist, eine Zone für optimales Sehen (23A) bzw. eine Zone für verschlechtertes Sehen (23B) des Sichtfeldes abgrenzt, für die die Person (10) angibt, auf optimale bzw. verschlechterte Weise das Testbild (22) des visuellen Tests durch die ophthalmologische Apparatur zur Sehkorrektur zu sehen, und
d) Aufzeichnen eines repräsentativen Grenzindikators für die Grenze (23) zwischen Zonen mit optimalem/verschlechtertem Sehen,
wobei:
- die Schritte a) bis d) mindestens ein erstes Mal mit einer Person (10) ausgeführt werden, die mit einer ersten ophthalmologischen Apparatur zur Sehkorrektur ausgestattet ist, um eine erste Grenze (23) zu bestimmen und einen ersten Grenzindikator aufzuzeichnen,
- die Person (10) tatsächlich oder virtuell mit einer zweiten ophthalmologischen Apparatur (14) zur visuellen Korrektur ausgestattet ist, die sich von der ersten ophthalmologischen Apparatur unterscheidet, eine zweite Grenze (24) von Zonen für optimales/verschlechtertes Sehen des Testbildes (22) durch die Person (10) auf der Anzeigefläche (21) in der Konfiguration, die sich aus den Schritten a) und b) ergibt, bestimmt wird, die zweite Grenze (24) eine zweite Zone für optimales Sehen (24A) bzw. eine zweite Zone für verschlechtertes Sehen (24B) des Sichtfeldes (FOV) abgrenzt, für die die Person (10), die tatsächlich oder virtuell mit einer zweiten ophthalmologischen Apparatur (14) ausgestattet ist, angibt oder vermutet wird, das Testbild (22) des visuellen Tests auf optimale bzw. verschlechterte Weise zu sehen,
- ein zweiter repräsentativer Grenzindikator für die zweite Grenze (24) zwischen Zonen mit optimalem/verschlechtertem Sehen aufgezeichnet wird,
- der erste Grenzindikator und der zweite Grenzindikator miteinander verglichen werden und
- aus dem vorausgehenden Vergleich eine optische Leistung der Person (10) unter den Betrachtungsbedingungen der Schritte a) und b) ermittelt wird.

2. Verfahren zum visuellen Testen nach Anspruch 1, wobei im Schritt d) der Grenzindikator der Position und/oder der Form der Grenze (23) entspricht.

3. Verfahren zum visuellen Testen nach Anspruch 1 oder 2, wobei die zweite Grenze (24) bestimmt wird, indem die Schritte a) bis c) für die Person (10), die mit einem tatsächlichen Exemplar der ophthalmologischen Apparatur (14) ausgestattet ist, ein zweites Mal durchgeführt werden.

4. Verfahren zum visuellen Testen nach Anspruch 1 oder 2, wobei die zweite Grenze (24) über eine digitale Simulation des Sichtfeldes (FOV) der Person (10) bestimmt wird, die virtuell mit der zweiten ophthalmologischen Apparatur (14) ausgestattet ist und das Testbild (22) unter denselben Bedingungen wie in den Schritten a) und b) betrachtet.

5. Verfahren zum visuellen Testen nach einem der Ansprüche 1 bis 4, das einen Schritt f) aufweist, in dem auf einer Nutzfläche der Anzeigefläche (21), die der Zone für optimales Sehen (23A) des Sichtfeldes der Person (10) entspricht, die mit der ophthalmologische Apparatur zur Sehkorrektur ausgestattet ist, ein ergänzender visueller Test angezeigt wird, der mindestens ein ergänzendes Testbild aufweist.

6. Verfahren zum visuellen Testen nach einem der Ansprüche 1 bis 4, wobei das Testbild (22), die erste Grenze (23) und die zweite Grenze (24) in Überlagerung angezeigt werden.

7. Verfahren zum visuellen Testen nach einem der Ansprüche 1 bis 6, wobei im Schritt c) die Grenze (23, 24) für eine vorgegebene Position des Kopfes (11) der Person (10) im Verhältnis zur Anzeigefläche (21) bestimmt wird.

8. Verfahren zum visuellen Testen nach einem der Ansprüche 1 bis 7, wobei im Schritt b) die Position des Testbildes (22) des visuellen Tests auf der Anzeigefläche (21) von der Position des Kopfes (11) der Person (10) im Verhältnis zur Anzeigefläche (21) abhängt.

9. Verfahren zum visuellen Testen nach einem der Ansprüche 1 bis 8, wobei im Schritt c) die Grenze (23, 24) zwischen Zonen mit optimalem/verschlechtertem Sehen von der Person (10) bestimmt wird, die die Grenze (23, 24) auf der Anzeigefläche (21) anlegt.

## Claims

1. A method for testing the vision of an individual (10) wearing a piece of vision-correcting ophthalmic equipment, said method including the following steps:
a) placing said individual (10) at a predetermined observation distance (D1) from a displaying area (21), at which said individual (10) observes, with a natural cephalic carriage, said displaying area (21),
b) displaying, on said displaying area (21), a visual test comprising at least one test image (22),
c) determining, in said displaying area (21), a border (23) of zones of optimal/degraded vision of said test image (22) by the individual (10) in the configuration resulting from steps a) and b), said border (23) respectively bounding, in a visual field of said individual (10) equipped with said piece of ophthalmic equipment, a zone of optimal vision (23A) and a zone of degraded vision (23B) of said visual field, for which zones said individual (10) indicates whether he respectively sees optimally or degradedly said test image (22) of the visual test through said piece of vision-correcting ophthalmic equipment, and
d) recording a border indicator representative of said border (23) of zones of optimal/degraded vision,
wherein:
- steps a) to d) are executed at least a first time, with an individual (10) equipped with a first piece of vision-correcting ophthalmic equipment, to determine a first border (23) and to record a first border indicator,
- the individual (10), actually or virtually being equipped with a second piece of vision-correcting ophthalmic equipment (14) that is different from said first piece of ophthalmic equipment, a second border (24) of zones of optimal/degraded vision of said test image (22) by said individual (10) is determined in said displaying area (21) in the configuration resulting from steps a) and b), said second border (24) respectively bounding a second zone of optimal vision (24A) and a second zone of degraded vision (24B) of said visual field (FOV), for which zones said individual (10) actually or virtually equipped with said second piece of ophthalmic equipment (14) indicates or is assumed to respectively see optimally or degradedly said test image (22) of the visual test,
- a second border indicator representative of said second border (24) of zones of optimal/degraded vision is recorded,
- said first border indicator and said second border indicator are compared, and
- from the preceding comparison an optical performance of the individual (10) under the observation conditions of steps a) and b) is deduced.

2. The vision-testing method according to claim 1, wherein, in step d), the border indicator corresponds to the position and/or shape of said border (23).

3. The vision-testing method according to claim 1 or 2, wherein said second border (24) is determined by executing a second time steps a) to c) for said individual (10) equipped with a real example of said second piece of ophthalmic equipment (14).

4. The vision-testing method according to claim 1 or 2, wherein said second border (23) is determined by numerical simulation of the visual field (FOV) of said individual (10) virtually equipped with said second piece of ophthalmic equipment (14) and observing the test image (22) under the same conditions as those of steps a) and b).

5. The vision-testing method according to anyone of claims 1 to 4, including a step f) in which, on a useful area of the displaying area (21) corresponding to the zone of optimal vision (23A) of the visual field of the individual (10) equipped with said piece of vision-correcting ophthalmic equipment, a complementary visual test comprising at least one complementary test image is displayed.

6. The vision-testing method according to anyone of claims 1 to 4, wherein the test image (22), the first border (23) and the second border (24) are displayed in superposition.

7. The vision-testing method according to anyone of claims 1 to 6, wherein, in step c), said border (23, 24) is determined for a given position of the head (11) of the individual (10) with respect to the displaying area (21).

8. The vision-testing method according to anyone of claims 1 to 7, wherein, in step b), the position of the test image (22) of said visual test in said displaying area (21) depends on the position of the head (11) of the individual (10) with respect to the displaying area (21).

9. The vision-testing method according to anyone of claims 1 to 8, wherein, in step c), said border (23, 24) of zones of optimal/degraded vision is determined by the individual (10) who draws said border (23, 24) in said displaying area (21).
